(19) Europäisches Patentamt
European Patent Office
Office européen des brevets

(11) Veröffentlichungsnummer: **0 019 750**
**A1**

(12) # EUROPÄISCHE PATENTANMELDUNG

(21) Anmeldenummer: **80102445.6**

(22) Anmeldetag: **06.05.80**

(51) Int. Cl.³: **C 07 C 121/38**

(30) Priorität: **09.05.79 DE 2918609**

(43) Veröffentlichungstag der Anmeldung: **10.12.80**
**Patentblatt 80/25**

(84) Benannte Vertragsstaaten: **CH DE FR GB IT LI NL**

(71) Anmelder: **HOECHST AKTIENGESELLSCHAFT, Zentrale Patentabteilung Postfach 80 03 20, D-6230 Frankfurt/Main 80 (DE)**

(72) Erfinder: **Finke, Manfred, Dr., Behringstrasse 25, D-6233 Kelkheim (Taunus) (DE)**
Erfinder: **Mündnich, Rainer, Dr., Schreyerstrasse 11, D-6000 Frankfurt am Main 70 (DE)**

(54) **Verfahren zur Herstellung von Cyanhydrinacylaten Alpha, Beta-ungesättigter Aldehyde.**

(57) Cyanhydrinacylate α,β-ungesättigter Aldehyde, z.B. Acroleincyanhydrinacetat, werden in guter Ausbeute und Reinheit hergestellt durch Umsetzung von Cyanhydrinen α,β-ungesättigter Aldehyde mit organischen Säurehydriden in Gegenwart von anorganischen und/oder organischen Säuren, insbesondere mit $P_{Ka}$-Werten unterhalb von etwa 2,5 als Katalysator. Die Verfahrensprodukte sind Zwischenprodukte auf verschiedenen Sachgebieten, vor allem auf dem Pharmagebiet zur Herstellung biologisch aktiver Substanzen.

EP 0 019 750 A1

BEZEICHNUNG GEÄNDERT
siehe Titelseite





0019750

Verfahren zur Herstellung von Cyanhydrinacylaten α,ß-ungesättigter Aldehyde

Cyanhydrinacylate α,ß-ungesättigter Aldehyde sind wertvolle Zwischenprodukte auf verschiedenen Sachgebieten, insbesondere auf dem Pharmagebiet zur Herstellung biologisch aktiver Substanzen.

Eine gängige Methode zur Herstellung des wohl bekanntesten Cyanhydrinacylates eines α,ß-ungesättigten Aldehyds - nämlich des Acroleincyanhydrinacetats - besteht in der Umsetzung von Acroleincyanhydrin mit überschüssigem Acetanhydrid in Gegenwart von etwa 50 Mol-% Natriumacetat /Van Sleen, Rec.Trav.Chem. P.-B. 21, S. 209 ff., insbesondere S. 215 (1902)7; aus den mitgeteilten Zahlenwerten errechnet sich eine Ausbeute von etwa 33 % d.Th.

R.Rambaud /Bull.soc.chim. 1, S. 1317 ff, insbesondere S. 1326/27 (1934)7 gibt an, nach derselben Methode eine Ausbeute von 70 % d.Th. an Acroleincyanhydrinacetat erhalten zu haben.

Die ziemlich aufwendige Aufarbeitung des Reaktionsgemisches (s. Van Sleen, a.a.O.) läßt die Methode jedoch für die technische Durchführung wenig geeignet erscheinen; denn das Acroleincyanhydrinacetat muß aus dem Reaktionsgemisch nach der Zersetzung des überschüssigen Acetanhydrids - das daher nicht in den Prozeß zurückgeführt werden kann - mit Äther extrahiert, die erhaltene organische Phase mit Natriumcarbonatlösung neutralisiert, über wasserfreiem Natriumsulfat getrocknet, filtriert, eingeengt und schließlich durch Vakuumdestillation weiter gereinigt werden.

Außerdem besitzt das so hergestellte Acroleincyanhydrinacetat - wie eigene Versuche ergeben haben - eine (gaschromatographisch bestimmte) Reinheit von bestenfalls nur etwa 83 %, so daß wohl auch Rambaud kaum mehr als 58 % d.Th. an reinem Acroleincyanhydrinacetat erhalten haben kann.

Wegen der ungenügenden Reinheit des bei dem bekannten Verfahren anfallenden Produkts kann dieses für eine Reihe von Umsetzungen wie z.B. für Radikalreaktionen, bei denen schon geringfügige Verunreinigungen erheblich stören, nicht ohne eine zusätzliche Reinigung verwendet werden.

Es bestand daher die Aufgabe, ein verbessertes Verfahren zur Herstellung von Acroleincyanhydrinacetat sowie auch von anderen Cyanhydrinacylaten α,ß-ungesättigter Aldehyde zu finden, welches sich auch in technischem Maßstab wirtschaftlich durchführen läßt und ein reineres Produkt liefert.

Diese Aufgabe konnte erfindungsgemäß in einfacher und befriedigender Weise durch die Verwendung von anorganischen und/oder organischen Säuren als Katalysatoren für die Umsetzung von Cyanhydrinen α,ß-ungesättigter Aldehyde mit organischen Säureanhydriden gelöst werden.

Erfindungsgegenstand ist somit ein Verfahren zur Herstellung von Cyanhydrinacylaten α,ß-ungesättigter Aldehyde durch Umsetzung von Cyanhydrinen α,ß-ungesättigter Aldehyde mit organischen Säureanhydriden in Gegenwart eines Katalysators, das dadurch gekennzeichnet ist, daß man als Katalysator anorganische und/oder organische Säuren verwendet.

Das ausgezeichnete Gelingen und das Ausbleiben nennenswerter Nebenreaktionen bei dieser säurekatalysierten Umsetzung der sehr empfindlichen Ausgangs-Cyanhydrine in Abwesenheit von Natriumacetat, und die Entstehung eines

reinen Endprodukts in guten bis sehr guten Ausbeuten war außerordentlich überraschend, weil nämlich die dem Acroleincyanhydrin I

$$CH_2 = CH - \underset{OH}{CH} - CN \qquad (I)$$

hinsichtlich der Reaktivität vergleichbare 2-Hydroxy-buten(3)-säure II

$$CH_2 = CH - \underset{OH}{CH} - COOH \qquad (II)$$

mit Acetanhydrid in Gegenwart von Säure (Schwefelsäure) nur eine schlechte Ausbeute an Acylierungsprodukt liefert, mit Acetanhydrid/Natriumacetat jedoch glatt in das entsprechende Acylierungsprodukt umgewandelt werden kann /R. Rambaud, Bull.soc.chim. 1, S. 1327 (1934)/.

Als Ausgangsstoffe für das erfindungsgemäße Verfahren kommen vorzugsweise solche Cyanhydrine α,ß-ungesättigter Aldehyde in Frage, welche sich vom Acrolein ableiten und unter die folgende Formel III fallen:

$$CH_2 = \underset{R^1}{C} - \underset{OH}{CH} - CN \qquad (III),$$

worin $R^1$ = H,
$(C_1-C_4)$-Alkyl,
Phenyl oder Benzyl,
vorzugsweise =H, $CH_3$ oder $C_2H_5$, insbesondere =H.

Unter diese Formel fallende Cyanhydrine sind also z.B.: Acrolein-, α-Methacrolein-, α-Äthacrolein-, α-n-Butylacrolein-, α-Phenylacrolein-, α-Benzylacrolein-cyanhydrin etc.

Die Cyanhydrine lassen sich nach bekannten Methoden - wie etwa beschrieben in der JP.-OS 71 18733 - herstellen.

Als organische Säureanhydrid-Ausgangsmaterialien für das erfindungsgemäße Verfahren eignen sich bevorzugt Essigsäure- und Propionsäureanhydrid, insbesondere Essigsäureanhydrid.

Das Molverhältnis von Cyanhydrin zu dem Acylierungsmittel (organisches Säureanhydrid) beträgt zweckmäßig etwa 1:1 bis etwa 1:1,5. Größere Überschüsse an Acylierungsmittel sind möglich, bringen jedoch keine Vorteile.

Katalysatoren für das erfindungsgemäße Verfahren sind anorganische und/oder organische Säuren, hauptsächlich solche mit $p_{Ka}$-Werten bis zu etwa 5, insbesondere bis zu etwa 2,5. Anorganische und organische Säuren mit $p_{Ka}$-Werten zwischen etwa 2,5 und etwa 5 sind z.B. Essigsäure, Propionsäure und Buttersäure; bevorzugte Säuren mit $p_{Ka}$-Werten unter etwa 2,5 sind z.B. Chlorwasserstoffsäure, Bromwasserstoffsäure, Jodwasserstoffsäure, Schwefelsäure, Salpetersäure, Perchlorsäure, Chlorsulfonsäure, Phosphorsäure, Dichloressigsäure, Trichloressigsäure, Trifluoressigsäure, p-Toluolsulfonsäure etc. Besonders bevorzugte Säuren sind Schwefelsäure und/oder p-Toluolsulfonsäure.

Die schwächeren Säuren mit $p_{Ka}$-Werten zwischen etwa 2,5 und etwa 5 werden in einer Menge von etwa 1 bis 10 Mol/Mol Cyanhydrin-Ausgangsmaterial, die stärkeren Säuren mit $p_{Ka}$-Werten unter etwa 2,5 in einer Menge von etwa 0,001 bis 0,1 Mol/Mol Cyanhydrin-Ausgangsmaterial, eingesetzt.

Es kann sowohl eine der genannten Säuren allein als auch eine Mischung von zwei oder mehreren dieser Säuren verwendet werden.

Das Verfahren wird im allgemeinen so durchgeführt, daß man das organische Säureanhydrid in das vorgelegte ungesättigte

Cyanhydrin oder umgekehrt das Cyanhydrin in das organische Säureanhydrid eintropft. Die Katalysatoren können mit dem organischen Säureanhydrid oder mit dem Cyanhydrin oder mit beiden Reaktionspartnern vermischt werden.

Die Verwendung eines inerten Lösungsmittels wie z.B. Methylenchlorid, Chloroform, Dioxan, Tetrahydrofuran, Diisopropyläther, Pentan, Hexan, Cyclohexan, Benzol, Toluol etc. ist möglich; vorzugsweise wird jedoch in Abwesenheit eines solchen inerten Lösungsmittels gearbeitet.

Die Reaktion wird im allgemeinen in einem Temperaturbereich zwischen etwa 0 und 120°C, vorzugsweise zwischen etwa 20 und 80°C, und bei Atmosphärendruck durchgeführt; in manchen Fällen kann es aber auch zweckmäßig sein, bei erhöhtem Druck zu arbeiten.

Die Reaktionsdauer ist in weiten Grenzen variabel und beträgt je nach Temperatur, Druck, Versuchsanordnung und Größe des Ansatzes im allgemeinen zwischen etwa 0,5 und 10 Stunden.

Das Verfahren kann sowohl kontinuierlich als auch diskontinuierlich durchgeführt werden.

Nach dem Verfahren werden Cyanhydrinacylate α,ß-ungesättigter Aldehyde in guten bis sehr guten Ausbeuten erhalten. Wenn man von Cyanhydrinen der Formel III sowie von Essigsäure- oder Propionsäureanhydrid ausgeht, besitzen die erhaltenen Cyanhydrinacylate die Formel IV:

$$CH_2 = \underset{R^1}{C} - \underset{OR^2}{CH} - CN \qquad (IV)$$

worin $R^1$ die gleiche Bedeutung wie in Formel III besitzt und

$R^2 = COCH_3$ oder $COC_2H_5$.

Nicht verbrauchte Anteile des Acylierungsmittels (organische Säureanhydride) und die dem Anhydrid entsprechende Carbonsäure können durch Destillation zurückgewonnen und ohne weitere Reinigung erneut in das Verfahren eingesetzt werden. Die meist flüssigen Reaktionsprodukte können aus dem Reaktionsgemisch ohne vorhergehende Neutralisation direkt herausdestilliert werden und fallen in hoher Reinheit an.

Das Verfahren stellt daher einen erheblichen Fortschritt dar.

Die Verfahrensprodukte sind Zwischenprodukte, hauptsächlich zur Herstellung von Flammhemmitteln oder biologisch aktiven Verbindungen mit bakterizider, fungizider oder herbizider Wirkung dar. Derartige biologisch aktive Verbindungen werden etwa erhalten durch Umsetzung der Verfahrensprodukte mit P-H-Verbindungen der Formel V:

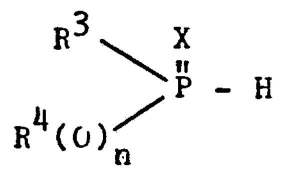

(V)

worin $R_3$ und $R_4$ = gegebenenfalls subst. organ. Reste,
X = Sauerstoff oder Schwefel, und
n = 0 oder 1

in Gegenwart katalytischer Mengen von Radikalbildnern zu Verbindungen der Formel VI:

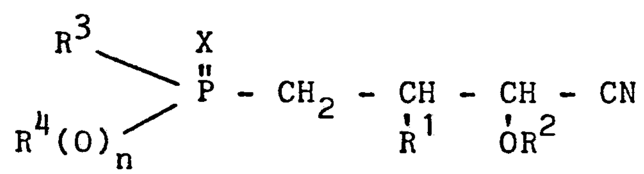

(VI)

(Bedeutung von $R^1$ und $R^2$ s. Formel IV, von $R^3$ und $R^4$ s. Formel V)

nach dem Verfahren der Patentanmeldung P 28 49 003.1 (HOE 78/F 245), Überführung dieser Verbindungen VI in die Aminonitrile und deren Verseifung. Man kommt so beispielsweise zu dem Hemmstoff für Glutaminsynthetase, Phosphinothricin

$$CH_3-\overset{O}{\overset{\|}{\underset{OH}{P}}}-CH_2-CH_2-\underset{NH_2}{CH}-COOH \qquad (VII)$$

/‾Helv.Chim.Acta 55, S. 224 - 39 (1972)_7.

Von dieser und ähnlichen Verbindungen ist auch eine herbizide Wirkung bekannt (DE-OS 27 17 440).

Die Erfindung wird nun durch die nachfolgenden Beispiele näher erläutert.

Beispiel 1:

Acroleincyanhydrinacetat

In 415 g (5 Mol) frisch hergestelltes, rohes Acroleincyanhydrin, das 7,2 g p-Toluolsulfonsäure enthält, wird bei 25 - 30°C ein Gemisch aus 612 g (6 Mol) Acetanhydrid, 50 ml Eisessig und 5 g p-Toluolsulfonsäure eingetropft. Nach beendetem Zutropfen wird 30 Min. bei 60°C nachgerührt, und anschließend die Reaktionslösung unter vermindertem Druck fraktioniert destilliert.
Man erhält 575 g Acroleincyanhydrinacetat ($K_{p_{18\ mbar}}$ : 74 - 76°). Die Reinheit des Acroleincyanhydrinacetats beträgt ca. 94 % (GC).

Aus einer Zwischenfraktion lassen sich nochmals 15 g Produkt isolieren, so daß die Gesamtausbeute an Acroleincyanhydrinacetat 554 g (89 % d.Th.) beträgt.

Beispiel 2:

Acroleincyanhydrinacetat

In 249 g (3 Mol) rohes Acroleincyanhydrin, vermischt mit 200 ml Eisessig und 3 g p-Toluolsulfonsäure wird bei 20 - 25°C 340 g (3,3 Mol) Acetanhydrid eingetropft. Nach Beendigung der exothermen Reaktion rührt man noch 2 Stdn. bei 70°C nach. Anschließend wird zunächst Essigsäure und überschüssiges Acetanhydrid unter Wasserstrahlvakuum abdestilliert und danach der Rückstand unter vermindertem Druck fraktioniert.
Bei $K_{27\ mbar}$: 81°C destillieren 326 g dünnschichtchromatographisch einheitliches Acroleinhydrinacetat (87 % d.Th.).

Beispiel 3:

Acroleincyanhydrinacetat

In ein Gemisch aus 120 g (1,18 Mol) Acetanhydrid und 60 g (1 Mol) Eisessig werden bei 40°C 83 g (1 Mol) rohes Acroleincyanhydrin eingetropft. Nach beendetem Zutropfen rührt man noch 3 Stdn. bei 70 - 80° nach. Anschließend wird zunächst Essigsäure und überschüssiges Acetanhydrid unter Wasserstrahlvakuum abdestilliert, und danach der Rückstand unter vermindertem Druck fraktioniert.
Bei $K_{p\,16\ mbar}$: 70 - 71°C destillieren 100 g dünnschichtgromatographisch einheitliches Acroleincyanhydrinacetat (80 % d.Th.).

Beispiel 4:

α-Äthacroleincyanhydrinacetat

In ein Gemisch aus 120 g (1,18 Mol) Acetanhydrid, 60 g

(1 Mol) Eisessig und 1 g p-Toluolsulfonsäure werden bei 50°C 111 g (1 Mol) rohes α-Äthacroleincyanhydrin eingetropft. Nach beendetem Zutropfen rührt man noch 3 Stdn. bei 80°C nach. Anschließend wird zunächst Essigsäure und überschüssiges Acetanhydrid unter Wasserstrahlvakuum abdestilliert, und danach der Rückstand unter Hochvakuum fraktioniert. Bei $K_{p18\ mbar}$: 91 - 93°C destillieren 142 g dünnschichtchromatographisch einheitliches α-Äthacroleincyanhydrinacetat (93 % d.Th.).

Patentansprüche:

1. Verfahren zur Herstellung von Cyanhydrinacylaten α,ß-ungesättigter Aldehyde durch Umsetzung von Cyanhydrinen α,ß-ungesättigter Aldehyde mit organischen Säureanhydriden in Gegenwart eines Katalysators, dadurch gekennzeichnet, daß man als Katalysator anorganische und/oder organische Säuren verwendet.

2. Verfahren nach Anspruch 1, dadurch gekennzeichnet, daß man als Cyanhydrine α,ß-ungesättigter Aldehyde Verbindungen der Formel III

$$CH_2 = \underset{R^1}{\underset{|}{C}} - \underset{OH}{\underset{|}{CH}} - CN \qquad (III),$$

worin $R^1$ = H,
(C$_1$-C$_4$)-Alkyl,
Phenyl oder Benzyl,
vorzugsweise =H, $CH_3$ oder $C_2H_5$, insbesondere =H,

verwendet.

3. Verfahren nach Ansprüchen 1 bis 2, dadurch gekennzeichnet, daß man als organische Säureanhydride Essigsäure- oder Propionsäureanhydrid, vorzugsweise Essigsäureanhydrid verwendet.

4. Verfahren nach den Ansprüchen 1 bis 3, dadurch gekennzeichnet, daß man als Katalysatoren solche anorganischen und/oder organischen Säuren verwendet, welche einen $p_{Ka}$-Wert unterhalb von etwa 2,5 besitzen.

5. Verfahren nach den Ansprüchen 1 bis 4, dadurch gekennzeichnet, daß man als Katalysatoren Schwefelsäure und/oder p-Toluolsulfonsäure verwendet.

6. Verfahren nach den Ansprüchen 1 bis 5, dadurch gekennzeichnet, daß man die Katalysatoren in einer Menge von etwa 0,001 bis 0,1 Mol/Mol Ausgangs-Cyanhydrin verwendet.

7. Verfahren nach den Ansprüchen 1 bis 6, dadurch gekennzeichnet, daß man die Umsetzung bei Temperaturen zwischen etwa 0 und 120°C, vorzugsweise zwischen etwa 20 und 80°C, durchführt.

Europäisches Patentamt

# EUROPÄISCHER RECHERCHENBERICHT

0019750 Nummer der Anmeldung

EP 80102445.6

| EINSCHLÄGIGE DOKUMENTE | | | KLASSIFIKATION DER ANMELDUNG (Int.Cl. 3) |
|---|---|---|---|
| Kategorie | Kennzeichnung des Dokuments mit Angabe, soweit erforderlich, der maßgeblichen Teile | betrifft Anspruch | |
| X | DE - B - 1 175 221 (KNAPSACK-GRIESHEIM AG)<br>* Beispiel 1; Zeilen 4,5 *<br>-- | 1,3,4,6,7 | C 07 C 121/38 |
| | US - A - 2 476 026 (CLIFFORD & LONG)<br>* Spalte 1, Zeilen 38-40 *<br>-- | 1,3-7 | |
| | US - A - 2 396 292 (SLOTTERBECK)<br>* Seite 1, rechte Spalte; Zeilen 21-28; Seite 2, Tabelle I *<br>---- | 1,4-7 | |

RECHERCHIERTE SACHGEBIETE (Int. Cl. 3)

C 07 C 121/00

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt

| Recherchenort | Abschlußdatum der Recherche | Prüfer |
|---|---|---|
| WIEN | 10-07-1980 | DASCHL |

EPA form 1503.1 06.78